# FASCICULE DE BREVET EUROPEEN

(11) **EP 2 545 030 B1**
(45) Date de publication et mention de la délivrance du brevet: **05.12.2018**
(21) Numéro de dépôt: 11707425.2
(22) Date de dépôt: 11.03.2011
(51) Int. Cl.: H01L 51/46, C07C 261/04, C07C 313/00, C07C 255/42

(54) **SOLUTIONS DE MATERIAUX MOLECULAIRES CONDUCTEURS ET MATERIAUX COMPOSITES A PARTIR DE CES SOLUTIONS**
LÖSUNGEN VON LEITENDEM MOLEKULAREM MATERIAL UND DARAUS HERGESTELLTE KOMPOSITMATERIALIEN
SOLUTIONS OF CONDUCTIVE MOLECULAR MATERIALS AND COMPOSITE MATERIALS PRODUCED FROM THESE SOLUTIONS

(30) Priorité: 12.03.2010 FR 1001003
(43) Date de publication de la demande: 16.01.2013
(73) Titulaire: THALES, 92400 Courbevoie (FR); Centre National d'Etudes Spatiales (C.N.E.S.), 75001 Paris (FR); Centre National de la Recherche Scientifique, 75016 Paris (FR)
(72) Inventeur: SOUQUE, Matthieu, F-31000 Toulouse (FR); VENDIER, Olivier, F-31120 Lacroix-falgarde (FR); VALADE, Lydie, F-31270 Cugnaux (FR); DE CARO, Dominique, F-31400 Toulouse (FR); DESMARRES, Jean-Michel, F-31280 Dremil-lafage (FR); COURTADE, Frédéric, F-31320 Castanet (FR)
(74) Mandataire: Esselin, Sophie
(86) Numéro de dépôt international: PCT/EP2011/053662
(87) Numéro de publication internationale: WO 2011/110653

(56) Documents cités:
- JP-A- 63 185 978
- I. SONDI, O. SIIMAN, E. MATIJEVIC: "Sythesis of Cdse nanoparticles in the presence of aminodextran as stabilizing and capping agent", J. COLLOID INTERFACE SCI., vol. 275, 19 mars 2004 (2004-03-19), pages 503-507, XP002632476,
- LIU Y ET AL: "Hydrothermal synthesis of square thin flake CdS by using surfactants and thiocarbohydrate", MATERIALS RESEARCH BULLETIN, ELSEVIER, KIDLINGTON, GB, vol. 36, no. 7-8, 1 mai 2001 (2001-05-01), pages 1231-1236, XP004240870, ISSN: 0025-5408, DOI: DOI:10.1016/S0025-5408(01)00614-6

## Description

Le domaine de l'invention est celui des matériaux moléculaires, pouvant trouver de nombreuses applications dans des domaines tels que les matériaux absorbants électromagnétiques ou les matériaux pour l'électronique (transistors, pistes, LED, stockage de données, etc.).

Plus précisément, de tels matériaux sont notamment recherchés pour leurs propriétés de conduction et magnétiques dans des applications spatiales, et plus précisément pour des absorbants RF à pertes diélectriques au-delà de 20 GHz. Pour cela il est nécessaire de disposer de matériaux répondant à l'ensemble des critères suivants :
- une conductivité comprise entre 1 et 1000 S.m⁻¹ au-delà de 20 GHz ;
- un dépôt possible sur des géométries complexes (angles saillants, etc.) ;
- une légèreté importante pour le matériel embarqué ;
- une épaisseur de dépôt contrôlable ;
- une bonne tenue en environnement spatial.

Il est actuellement connu des matériaux absorbants électromagnétiques comportant des composites à pertes électriques chargés en particules conductrices ou des composites à pertes magnétiques chargés en particules magnétiques. Ainsi il existe des absorbants large bande de type : mousses à faibles pertes électriques ou magnétiques (taux de charge en noir de carbone, carbure de silicium, ferrites, etc., inférieur à 1%). Néanmoins, ils donnent lieu à des épaisseurs importantes supérieures à environ 3 mm et nécessitant en outre une mise en oeuvre notamment par plaques collées sur le boîtier métallique.

Il a également été proposé de réaliser des absorbants résonnants à partir de matériaux composites à fortes pertes électriques ou à fortes pertes magnétiques qui utilisent des taux de charges plus importants que les matériaux absorbants électromagnétiques large bande. Les matériaux absorbants magnétiques résonnants présentent des problèmes de masse volumique (au moins supérieure à 3,6 g.cm⁻³) et de fréquence de coupure. En effet au-delà d'une certaine fréquence, le matériau absorbant réalisé avec de telles particules ne présente plus de propriétés absorbantes. On rencontre typiquement ce problème pour des fréquences supérieures à une centaine de GigaHertz.

Les matériaux absorbants résonnants électriques nécessitent une très grande maîtrise de la dispersion des charges : les particules utilisées (comme le noir de carbone) étant très conductrices, il est nécessaire d'avoir un taux de charge situé dans la zone de percolation du matériau et donc de maîtriser très finement les concentrations en taux de particules.

Dans ce contexte, la présente invention exploite le fait de pouvoir élaborer des matériaux à conductivités modérées afin d'éviter le problème d'un seuil de percolation trop abrupt et propose d'utiliser des matériaux moléculaires faciles à mettre en oeuvre.

De manière générale on définit des matériaux moléculaires par leur structure. Il s'agit plus précisément de matériaux constitués de briques moléculaires qui sont des molécules organiques ou des complexes de métaux de transition composés d'un ou plusieurs cations et de ligands organiques. La nature de ces briques et leur organisation permettent d'obtenir des matériaux ayant des propriétés physiques originales en terme notamment de conductivité, de ferromagnétisme ou bien encore de photochromisme.

Les conducteurs moléculaires les plus connus sont les polymères conducteurs. La conductivité de ces polymères dépend tout comme les matériaux semi-conducteurs, de leur niveau de dopage et de la quantité de charges libres présentes sur la chaîne. Un des inconvénients qu'ils présentent réside dans leur manque de stabilité au cours du temps, étant le siège de phénomène de neutralisation de charges (par exemple sous atmosphère humide ou sous exposition UV).

Il existe aussi des matériaux moléculaires conducteurs constitués uniquement de molécules organiques ou organo-métalliques (qui ne sont pas des polymères). Ces composés sont généralement obtenus sous forme de monocristaux qui ont intrinsèquement de hautes valeurs de conductivité (entre 1 et 100 000 S.m⁻¹) et conservent leurs propriétés électriques pendant plus de quinze ans. Ces matériaux sont assez friables et sont le plus fréquemment utilisés sous forme de dépôts. Ces matériaux sont très peu solubles et ces dépôts sur des surfaces se font généralement par électrodéposition, CVD, ou dépôts successifs de solutions de précurseurs. Toutes ces techniques ne permettent d'obtenir que des dépôts de faibles épaisseurs sur des surfaces réduites et sont plus ou moins rapides.

Le dépôt direct sur un substrat effectué par dépôt d'une solution « spin coating » ou par imprégnation «dip coating » ou bien encore par immersion dans une solution de conducteur moléculaire était jusqu'à présent très limité par les concentrations maximales très faibles des solutions : par exemple dans le DMSO, un précurseur tel que le TTF a une solubilité de 770 g/L, un précurseur tel que le TCNQ a une solubilité de 16 g/L et le composé moléculaire conducteur TTF·TCNQ a une solubilité de 0,8 g/L. Cette concentration ne permet pas de faire des dépôts directs, d'autant plus que pour ces très faibles concentrations une détérioration du composé peut se produire lors du séchage.

Il est connu du Document : I.Sondi, O. E.Matijevic, J.Colloid Interfac Sci.275 (2004 503-507) d'utiliser une molécule organique dans laquelle il existe une délocalisation électronique impliquant les groupements NH₂ et l'atome de sélénium, et une molécule d'aminodextrine. Il est également prévu du chlorure de cadmium(II), qui n'est ni organique, ni organo-métallique.

Il est également connu du Document : JP 63 185978 des matériaux conducteurs mais non élaborés sous la forme d'une solution stable. Ce Document décrit la synthèse de dérivés conducteurs du type [TTF][TCNQ-2,5-di-substitué] à bas point de fusion, permettant d'envisager la préparation de films.

Il est encore connu du Document de LIU ET AL « Hydrothermal synthesis of square thin flake CdS by using surfactants and thiocarbohydrate" d'utiliser le thiocarbohydrate (NH₂NHC(S)NHNH₂ avec un surfactant (tel que l'acide stéarique) et en présence du composé Cd(NO₃)₂ qui n'est ni organique, ni organo-métallique.

Pour pallier le problème de dissolution dans un solvant des matériaux moléculaires pré-cités, afin de faciliter notamment la fabrication de films, la présente invention apporte une solution au problème de l'obtention de solutions colloïdales stables à fortes concentrations en matériaux moléculaires. Sous cette forme la solution peut être directement déposée sur un substrat afin d'obtenir un film ou bien être associée à une solution précurseur d'une matrice pour obtenir des matériaux composites nanostructurés présentant une homogénéité optimale.

Plus précisément la présente invention a pour objet une solution stable en matériau moléculaire, comprenant :
- un premier type de molécules organiques ou organo-métalliques (M1) ;
- le premier type au moins présentant des groupements permettant une délocalisation électronique ;
- un troisième type de molécules (M3) pouvant être un agent tensio-actif ou un solvant,
   caractérisé en ce qu'elle comprend également un second type de molécules organiques ou organo-métalliques ou d'ions (M2) ;
- ledit troisième type étant capable de s'associer au premier ou au second types de molécules ou d'ions (M1, M2), pour former un adduit en solution dont la présence empêche la précipitation du composé M1-M2 formé par réaction entre M1 et M2 et permet d'obtenir une solution dont la concentration en composé M1-M2 est supérieure à celle régie par la constante de précipitation du composé M1-M2 en l'absence de molécules de troisième type M3 et ;
- les molécules dudit troisième type chargées de définir des adduits en solution avec les premier ou second types de molécules étant des molécules organo-solubles présentant au moins un groupe coordinant, pouvant être un composé appartenant à l'une des familles suivantes :
   - des amines pouvant être de la 1-octylamine ou de la 1-pentylamine ;
   - des acides carboxyliques ;
   - des esters ;
   - des siloxanes ;
   - des phosphines ;
   - des molécules possédant un système π coordinant pouvant être du 3-hexylthiophène.

La solution de la présente invention peut typiquement être stable en n'observant pas de précipitation pendant au minimum trois mois.

Selon une variante de l'invention, la solution comprend en outre un quatrième type de molécules M4, pouvant être un agent tensio-actif ou un solvant, capable de s'associer au premier et au second types de molécules organiques ou organo-métalliques.

Selon une variante de l'invention, les premier ou second types de molécules sont des molécules organiques ou organo-métalliques, des sels moléculaires organiques ou organo-métalliques.

Selon une variante de l'invention, le troisième et/ou le quatrième types de molécules est un composé comportant des fonctions de type amines ou acides carboxyliques ou phosphines ou molécules possédant un système π coordinant.

Selon une variante de l'invention, le premier et/ou le second type de molécules ou d'ions est une molécule organique ou organo-métallique de type donneur pouvant être de type :
- dérivé du TTF ;
- complexes de métaux de transition ;
- complexes de métaux à ligands bisdithiolènes : M(dmit)₂ à l'état neutre ou chargé avec M : Ni, Pd, Pt et dmit : le 4,5-dimercapto-1,3-dithiole-2-thione ;
- complexes de métaux à ligands de type TTF-bisdithiolène comme le tmdt : M(tmdt)₂ à l'état neutre ou chargé avec tmdt : le triméthylènetétrathiafulvalène-dithiolate et M : Ni, Pd, Pt ;
- complexes de métaux à ligands dcbdt : M(dcbdt)₂, à l'état neutre ou chargé avec dcbdt : dicyanobenzènedithiolate et M : Ni, Pd, Pt ;
- le pérylène.

Selon une variante de l'invention, le premier et/ou le second type de molécules est une molécule organique ou organo-métallique de type accepteur pouvant être de type :
- un dérivé du TCNQ : tétracyano-p-quinodiméthane ;
- le TNAP : le 11,11,12,12-tétracyanoaphto-2,6-quinodiméthane ;
- le DCNQI : la dicyanoquinonediimine.

Selon une variante de l'invention, lorsqu'un seul premier type ou second type est de type molécules organiques ou organo-métalliques, l'autre type peut être de type ionique et être compris parmi l'un des anions suivants : Hexafluorophosphates, Triiodure, Chlorure, Bromure, Arséniates, Antimoniates.

Selon une variante de l'invention, lorsqu'un seul premier type ou second type est de type molécule organique ou organo-métallique, l'autre type peut être de type ionique et être compris parmi l'un des cations suivants : Na⁺ (Sodium), K⁺ (Potassium) ou un cation de métaux de transition.

Selon une variante de l'invention :
- les molécules de premier type sont des molécules de TTF ;
- les molécules de second type sont des molécules de TCNQ ;
- les molécules de troisième type sont des molécules d'amines à longues chaînes carbonées.

Selon une variante de l'invention :
- les molécules de premier type sont des molécules de TTF ;
- les molécules de second type sont des molécules de TCNQ ;
- les molécules de troisième type sont des molécules d'amines à longues chaînes carbonées.

L'invention a aussi pour objet un procédé de préparation d'une solution stable en matériau moléculaire selon l'invention caractérisé en ce qu'il comporte le mélange d'un premier type de molécules organiques ou organo-métalliques M1 et d'un second type de molécules organiques ou organo-métalliques ou d'ions M2, le premier type au moins présentant des groupements permettant une délocalisation électronique, et étant susceptibles de former un cristal moléculaire, en présence d'au moins un troisième type de molécules M3 pouvant être un agent tensio-actif ou un solvant, capable de s'associer au premier ou au second types de molécules ou d'ions M1,M2, pour former un adduit en solution dont la présence empêche la précipitation du composé M1-M2 formé par réaction entre M1 et M2 et permet d'obtenir une solution dont la concentration en composé M1-M2 est supérieure à celle régie par la constante de précipitation du composé M1-M2 en l'absence de molécules de troisième type M3.

Selon une variante de l'invention, le procédé comporte l'ajout d'un troisième type de molécules M3 et d'un quatrième type de molécules M4 pouvant être un agent tensio-actif ou un solvant, capable de s'associer au premier et au second types de molécules organiques ou organo-métalliques M1, M2.

Selon une variante de l'invention, le procédé comprend les étapes suivantes :
- le mélange de molécules de TTF et de molécules d'amines à longues chaînes carbonées dans un solvant organique, de façon à obtenir un mélange primaire ;
- l'ajout des molécules de TCNQ dans un solvant organique, au mélange primaire.

Selon une variante de l'invention, le procédé comprend les étapes suivantes :
- le mélange de molécules de TTF₃(BF₄)₂ et de molécules d'amines à longues chaînes carbonées dans un solvant organique, de façon à obtenir un mélange primaire ;
- l'ajout de molécules de [(*n*-C₄H₉)₄N][Ni(dmit)₂] dans un solvant organique au mélange primaire.

L'invention a aussi pour objet un procédé de préparation d'une poudre nanoparticulaire de matériau moléculaire caractérisée en ce qu'elle est obtenue par séchage d'une solution stable en matériau moléculaire selon l'invention.

L'invention a aussi pour objet un procédé de fabrication d'un film à base de matériau moléculaire caractérisé en ce qu'il comporte le dépôt d'une solution stable en matériau moléculaire selon l'invention sur un support, puis le séchage de ladite solution.

L'invention a aussi pour objet un procédé de fabrication d'un matériau composite à base de matériau moléculaire caractérisé en ce qu'il comporte le mélange d'une solution stable selon l'invention, à une matrice organique ou inorganique.

L'invention a aussi pour objet un procédé de fabrication d'un matériau composite à base de matériau moléculaire caractérisé en ce qu'il comporte le mélange d'une poudre nanométrique obtenue par le procédé de l'invention, à une matrice organique ou inorganique.

L'invention a encore pour objet l'utilisation d'une solution stable de matériau moléculaire selon l'invention, pour la réalisation d'un matériau absorbant électromagnétique.

L'invention a encore pour objet l'utilisation d'une solution stable de matériau moléculaire selon l'invention, pour la réalisation d'un matériau actif pour l'électronique. Il peut notamment s'agir de piste conductrice ou de transistor ou de diode électroluminescente.

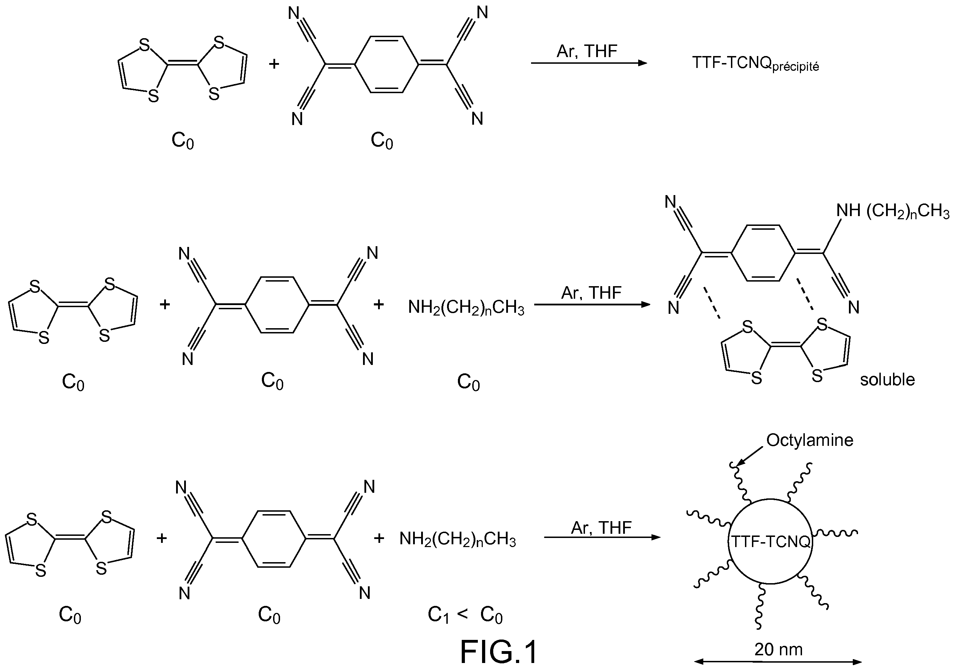

L'invention sera mieux comprise et d'autres avantages apparaîtront à la lecture de la description qui va suivre donnée à titre non limitatif et grâce à la figure 1 annexée relative à un exemple d'adduit formé dans une solution de matériaux moléculaires conducteurs de l'invention.

De manière générale, les premiers types moléculaires M1 et M2 jouent des rôles équivalents et peuvent être de type molécule organique ou organo-métallique simple, sel moléculaire ou sel inorganique. Pour satisfaire la présente invention, il est nécessaire d'employer en outre un troisième type moléculaire M3 pouvant être un agent tensio-actif ou même un solvant, capable de s'associer à l'un des types M1 ou M2, pour former un adduit en solution, un adduit correspondant de manière connue à une espèce formée par combinaison directe d'un type M1 et d'un type M3, ou d'un type M2 et d'un type M3.

En effet, la présente invention propose d'associer aux premier et second types de molécules M1 ou M2 un troisième type de molécules M3, qui permet d'éviter que dans un solvant la mise en présence de M1 et M2 provoque la précipitation du conducteur moléculaire insoluble M1-M2, la formation du solide précité pouvant résulter d'une réaction d'oxydoréduction ou d'une réaction de transfert de charge. La solution stable résultante de l'association de M1, M2 et M3 pourra alors contenir des adduits moléculaires comportant deux des trois espèces ou les trois espèces bien dispersées en solution ou des nanoparticules du composé M1-M2 stabilisées par M3.

Pour pallier ce problème dans le cadre de la présente invention, les molécules de type M1 ou M2 peuvent aussi être associées à un type de molécules M3 et à un type de molécules M4 pour conduire à deux adduits solubles.

Par ailleurs, les molécules de type M1 et les molécules de type M3 ou les molécules de type M2 et les molécules de type M3 doivent être solubles dans un même solvant. La solvatation peut résulter de l'interaction entre les molécules de type M1 et les molécules de type M3 ou les molécules de type M2 et les molécules de type M3.

Les solvants des adduits M1 et M3 et des molécules M2 ou les solvants des adduits M2 et M3 peuvent être identiques ou différents.

### Exemples de molécules de type M1 et/ou de type M2 :

1) Molécules de type donneur :
   1.1. Dérivés du TTF (Tétrathiafulvalène),
      MDT-TTF(Méthylènedithiotétrathiafulvalène),
      BMDT-TTF (Bis(méthylènedithio)tétrathiafulvalène),
      BPDT-TTF (Bis(propylènedithio)tétrathiafulvalène),
      DMET (Diméthyl(éthylènedithio)disélénadithiafulvalène),
      HMTSF (Hexaméthylènetétrasélénafulvalène),
      TMTTF (Tétraméthyltétrathiafulvalène),
      TMTSF (Tétraméthyltétrasélénafulvalène),
      BEDT-TTF (Bis(éthylènedithio)tétrathiafulvalène).
   1.2. Complexes de métaux de transition.
   1.3. Famille des complexes de métaux bisdithiolènes comme par exemple :
      M(dmit)₂ à l'état neutre ou chargé, avec M : Ni, Pd, Pt et dmit : le 4,5-dimercapto-1,3-dithiole-2-thione.
   1.4. Famille des complexes de métaux à ligands de type TTF-bisdithiolène comme par exemple tmdt : M(tmdt)₂ à l'état neutre ou chargé avec tmdt : le triméthylènetétrathiafulvalène-dithiolate et M : Ni, Pd, Pt.
   1.5. Famille des complexes de métaux à ligands dcbdt : M(dcbdt)₂ à l'état neutre ou chargé avec dcbdt : dicyanobenzènedithiolate et M : Ni, Pd, Pt.
   1.6. Le Pérylène
2) Molécules de type accepteur pouvant être :
   des dérivés du TCNQ : tétracyano-p-quinodiméthane ;
   le TNAP : le 11,11,12,12-tétracyanoaphto-2,6-quinodiméthane;
   le DCNQI : la dicyanoquinonediimine.

Dans le cas où la solution ne comporte qu'un premier type M1 ou M2, précité, l'autre entité peut être de type ionique et dans ce cas, lesdits ions peuvent être :
3) Des anions :
   PF₆⁻ (Hexafluorophosphates)
   I₃⁻ (Triiodure)
   Cl⁻ (Chlorure)
   Br⁻ (Bromure)
   AsF₆⁻ (Arséniates)
   SbF₆⁻ (Antimoniates)
4) Des cations
   Na⁺ (Sodium)
   K⁺ (Potassium)
   Cations de métaux de transition

### Exemples de molécules de type M3 et de type M4 :

De manière générale, les troisième et quatrième types de molécules chargées de définir des adduits en solution avec les premier ou second types de molécules peuvent être des organo-solubles présentant au moins un groupe coordinant. Il peut s'agir notamment de composés comportant les fonctions suivantes :
- des amines (exemple : 1-octylamine, 1-pentylamine) ;
- des acides carboxyliques ;
- des esters ;
- des siloxanes ;
- des phosphines ;
- des molécules possédant un système π coordinant (exemple : 3-hexylthiophène).

De manière générale, le protocole pour obtenir une solution colloïdale de conducteurs moléculaires est le suivant :
Il convient de choisir deux types de molécules constitutives qui, en présence l'une de l'autre forment spontanément le conducteur moléculaire. On prépare une solution contenant d'une part le premier type de molécules M1 contenant également un troisième type de molécules M3 de type tensio-actif (le tensio-actif peut-être le solvant lui-même) dans un réacteur.

On ajoute alors le second type de molécules M2 solubilisé dans un volume de solvant dans le milieu réactionnel sous agitation magnétique. La réaction a lieu à température ambiante. Les concentrations en réactifs et en tensio-actifs vont déterminer la taille des particules formées et leur stabilité en solution. Typiquement la taille des particules ainsi formées peut être de l'ordre de quelques nanomètres, voire quelques dizaines de nanomètres.

Ces solutions colloïdales peuvent être alors séchées pour obtenir une poudre nanoparticulaire de conducteur moléculaire qui peut-être ressolubilisée ou dispersée selon les solvants utilisés.

La facilité de mise en oeuvre qu'apportent ces solutions colloïdales est très grande. De telles solutions permettent une incorporation homogène dans des matrices et une déposition par des procédés tels que le spin coating (dépôt à la tournette) ou le spray impossibles selon les techniques de l'art connu en raison des faibles concentrations des solutions de conducteurs moléculaires.

En outre, la synthèse in situ évite d'avoir recours à des étapes séparées de synthèse et dispersion.

### Premier exemple de procédé de préparation de solution de comportant des matériaux moléculaires conducteurs :

A titre d'exemple, la figure 1 illustre le schéma de la synthèse de solutions colloïdales stables de TTF-TCNQ, matériau moléculaire qui a une conductivité de 190000 S.m⁻¹ sur monocristal et qui permet d'obtenir typiquement une solution de matériaux moléculaires selon l'invention.

On procède dans une première étape à l'élaboration d'un mélange primaire de molécules de type M1 de TTF (concentration comprise entre 0,1 et 10 g/L) et de molécules de type M3 d'amines à chaînes carbonées saturées ou insaturées (concentration comprise entre 0,1 et 20 g/L) dans un solvant organique. En parallèle on met des molécules de type M2 de TCNQ dans un autre solvant organique (pouvant être identique, concentration comprise entre 0,1 et 20 g/L). On ajoute ensuite la solution contenant M2 à celle contenant M1 pour obtenir la solution de l'invention. Cette solution peut être concentrée par évaporation sans précipitation.

La conductivité des films obtenus par dépôt direct est de 20 S.m⁻¹.

L'utilisation d'un nombre inférieur d'équivalents d'amine permet d'obtenir des nanofils et des nanoparticules stables en solution.

### Deuxième exemple de procédé de préparation de solution de comportant des matériaux moléculaires conducteurs :

On procède au mélange de molécules de premier type M1 de TTF₃(BF₄)₂ (concentration comprise entre 0,1 et 10 g/L) avec des molécules de troisième type d'amine à longue chaîne carbonée, saturée ou insaturée entre 0,1 et 10 g/L) dans un solvant organique. Ce mélange est additionné à une solution dans un autre solvant organique des molécules de second type de [(n-C₄H₉)₄N][Ni(dmit)₂] (concentration entre 0,1 et 10 g/L) pour obtenir la solution de l'invention.

La conductivité des films obtenus par dépôt direct est de 200 S.m⁻¹.

## Revendications

1. Solution stable en matériau moléculaire, comprenant :
- un premier type de molécules organiques ou organo-métalliques (M1) ;
- le premier type au moins présentant des groupements permettant une délocalisation électronique ;
- un troisième type de molécules (M3) pouvant être un agent tensio-actif ou un solvant,
**caractérisé en ce qu'**elle comprend également un second type de molécules organiques ou organo-métalliques ou d'ions (M2) ;
- ledit troisième type étant capable de s'associer au premier ou au second types de molécules ou d'ions (M1, M2), pour former un adduit en solution dont la présence empêche la précipitation du composé M1-M2 formé par réaction entre M1 et M2 et permet d'obtenir une solution dont la concentration en composé M1-M2 est supérieure à celle régie par la constante de précipitation du composé M1-M2 en l'absence de molécules de troisième type M3 et ;
- les molécules dudit troisième type chargées de définir des adduits en solution avec les premier ou second types de molécules étant des molécules organo-solubles présentant au moins un groupe coordinant, pouvant être un composé appartenant à l'une des familles suivantes :
- des amines pouvant être de la 1-octylamine ou de la 1-pentylamine ;
- des acides carboxyliques ;
- des esters ;
- des siloxanes ;
- des phosphines ;
- des molécules possédant un système π coordinant pouvant être du 3-hexylthiophène.

2. Solution stable en matériau moléculaire selon la revendication 1, **caractérisée en ce qu'**elle comprend en outre :
- un quatrième type de molécules (M4) pouvant être un agent tensio-actif ou un solvant, capable de s'associer au premier et au second types de molécules organiques ou organo-métalliques (M1, M2) ;
- les molécules (M4) dudit quatrième type étant des molécules organo-solubles présentant au moins un groupe coordinant, pouvant être un composé appartenant à l'une des familles suivantes :
- des amines pouvant être de la 1-octylamine ou de la 1-pentylamine ;
- des acides carboxyliques ;
- des esters ;
- des siloxanes ;
- des phosphines ;
- des molécules possédant un système π coordinant pouvant être du 3-hexylthiophène.

3. Solution stable en matériau moléculaire selon l'une des revendications 1 ou 2, **caractérisée en ce que** les premier ou second types de molécules sont des molécules organiques ou organo-métalliques, des sels moléculaires organiques ou organo-métalliques.

4. Solution stable en matériau moléculaire selon l'une des revendications précédentes, **caractérisée en ce que** le premier et/ou le second type de molécules ou d'ions (M1, M2) est une molécule organique ou organo-métallique de type donneur pouvant être de type :
- dérivé du Tetrathiafulvalene [TTF] ;
- complexes de métaux de transition ;
- complexes de métaux à ligands bisdithiolènes : M(dmit)₂ à l'état neutre ou chargé avec M : Ni, Pd, Pt et dmit : le 4,5-dimercapto-1,3-dithiole-2-thione ;
- complexes de métaux à ligands de type TTF-bisdithiolène comme le tmdt : M(tmdt)₂ à l'état neutre ou chargé avec tmdt : le triméthylènetétrathiafulvalène-dithiolate et M : Ni, Pd, Pt ;
- complexes de métaux à ligands dcbdt : M(dcbdt)₂, à l'état neutre ou chargé avec dcbdt : dicyanobenzènedithiolate et M : Ni, Pd, Pt ;
- le pérylène.

5. Solution stable en matériau moléculaire selon l'une des revendications précédentes, **caractérisée en ce que** le premier et/ou le second type de molécules (M1, M2) est une molécule organique ou organo-métallique de type accepteur pouvant être de type :
- un dérivé du TCNQ : tétracyano-p-quinodiméthane ;
- le TNAP : le 11,11,12,12-tétracyanoaphto-2,6-quinodiméthane;
- le DCNQI : la dicyanoquinonediimine.

6. Solution stable en matériau moléculaire selon l'une des revendications précédentes, **caractérisée en ce que** lorsqu'un seul premier type ou second type est de type molécules organiques ou organo-métalliques, l'autre type peut être de type ionique et être compris parmi l'un des anions suivants : Hexafluorophosphates, Triiodure, Chlorure, Bromure, Arséniates, Antimoniates.

7. Solution stable en matériau moléculaire selon l'une des revendications précédentes, **caractérisée en ce que** lorsqu'un seul premier type ou second type est de type molécule organique ou organo-métallique, l'autre type peut être de type ionique et être compris parmi l'un des cations suivants : Na⁺ (Sodium), K⁺ (Potassium) ou un cation de métaux de transition.

8. Solution stable en matériau moléculaire selon l'une des revendications précédentes, **caractérisée en ce que**
- les molécules de premier type sont des molécules de TTF ;
- les molécules de second type sont des molécules de TCNQ ;
- les molécules de troisième type sont des molécules d'amines à longues chaînes carbonées.

9. Solution stable en matériau moléculaire selon l'une des revendications précédentes **caractérisée en ce que**
- les molécules de premier type sont des molécules de TTF ;
- les molécules de second type sont des molécules de TCNQ ;
- les molécules de troisième type sont des molécules d'amines à longues chaînes carbonées.

10. Procédé de préparation d'une solution stable en matériau moléculaire selon l'une des revendications précédentes **caractérisé en ce qu'**il comporte le mélange :
- d'un premier type de molécules organiques ou organo-métalliques (M1) et d'un second type de molécules organiques ou organo-métalliques ou d'ions (M2), le premier type au moins présentant des groupements permettant une délocalisation électronique, et étant susceptibles de former un cristal moléculaire, en présence d'au moins un troisième type de molécules (M3) ;
- les molécules dudit troisième type chargées de définir des adduits en solution avec les premier ou second types de molécules étant des molécules organo-solubles présentant au moins un groupe coordinant, pouvant être un composé appartenant à l'une des familles suivantes :
- des amines pouvant être de la 1-octylamine ou de la 1-pentylamine ;
- des acides carboxyliques ;
- des esters ;
- des siloxanes ;
- des phosphines ;
- des molécules possédant un système π coordinant pouvant être du 3-hexylthiophène et
dont la présence empêche la précipitation du composé M1-M2 formé par réaction entre M1 et M2 et permet d'obtenir une solution dont la concentration en composé M1-M2 est supérieure à celle régie par la constante de précipitation du composé M1-M2 en l'absence de molécules de troisième type M3.

11. Procédé de préparation d'une solution stable en matériau moléculaire comportant des matériaux moléculaires selon la revendication 10, **caractérisé en ce qu'**il comporte l'ajout d'un troisième type de molécules (M3) et d'un quatrième type de molécules (M4) pouvant être un agent tensio-actif ou un solvant, capable de s'associer au premier et au second types de molécules organiques ou organo-métalliques (M1, M2) ;
- les molécules (M4) dudit quatrième type étant des molécules organo-solubles présentant au moins un groupe coordinant, pouvant être un composé appartenant à l'une des familles suivantes :
- des amines pouvant être de la 1-octylamine ou de la 1-pentylamine ;
- des acides carboxyliques ;
- des esters ;
- des siloxanes ;
- des phosphines ;
- des molécules possédant un système π coordinant pouvant être du 3-hexylthiophène.

12. Procédé de préparation d'une solution stable en matériau moléculaire selon l'une des revendications précédentes, **caractérisé en ce qu'**il comprend les étapes suivantes :
- le mélange de molécules de TTF et de molécules d'amines à longues chaînes carbonées dans un solvant organique, de façon à obtenir un mélange primaire ;
- l'ajout de molécules de TCNQ dans un solvant organique, au mélange primaire.

13. Procédé de préparation d'une solution stable en matériau moléculaire selon l'une des revendications précédentes, **caractérisé en ce qu'**il comprend les étapes suivantes :
- le mélange de molécules de TTF₃(BF₄)₂ et de molécules d'amines à longues chaînes carbonées dans un solvant organique, de façon à obtenir un mélange primaire ;
- l'ajout de molécules de [(*n*-C₄H₉)₄N][Ni(dmit)₂] dans un solvant organique au mélange primaire.

14. Procédé de préparation d'une poudre nano-particulaire de matériau moléculaire **caractérisée en ce qu'**il comprend :
- les étapes de procédé de réalisation d'une solution stable selon l'une des revendications 10 à 13 ;
- une étape de séchage de ladite solution stable.

15. Procédé de fabrication d'un film à base de matériau moléculaire **caractérisé en ce qu'**il comprend :
- les étapes de procédé de réalisation d'une solution stable selon l'une des revendications 10 à 13 ;
- le dépôt de ladite solution stable sur un support ;
- le séchage de ladite solution stable sur ledit support.

16. Procédé de fabrication d'un matériau composite à base de matériau moléculaire **caractérisé en ce qu'**il comprend :
- les étapes de procédé de réalisation d'une solution stable selon l'une des revendications 10 à 13 ;
- le mélange de ladite solution stable à une matrice organique ou inorganique.

17. Procédé de fabrication d'un matériau composite à base de matériau moléculaire **caractérisé en ce qu'**il comprend :
- les étapes de préparation d'une poudre nano-particulaire de matériau moléculaire selon la revendication 15 ;
- le mélange de ladite poudre à une matrice organique ou inorganique.

18. Utilisation d'une solution stable de matériau moléculaire selon l'une des revendications 1 à 9, pour la réalisation d'un matériau absorbant électromagnétique.

19. Utilisation d'une solution stable de matériau moléculaire selon l'une des revendications 1 à 9, pour la réalisation d'un matériau actif pour l'électronique.

20. Utilisation d'une solution stable de matériau moléculaire selon la revendication 19, pour la réalisation de piste conductrice ou de transistor ou de diode électroluminescente.

## Patentansprüche

1. Stabile Lösung aus molekularem Material, umfassend:
- eine erste Art von organischen oder metallorganischen Molekülen (M1);
- wobei der erste Typ mindestens Gruppierungen aufweist, die eine elektronische Verlagerung ermöglichen;
- eine dritte Art von Molekülen (M3), die ein Tensid oder ein Lösungsmittel sein können,
**dadurch gekennzeichnet, dass** sie auch eine zweite Art von organischen oder metallorganischen Molekülen oder Ionen (M2) umfasst;
- wobei der dritte Typ in der Lage ist, sich mit der ersten oder zweiten Art von Molekülen oder Ionen (M1, M2) zu assoziieren, um ein Addukt in Lösung zu bilden, dessen Vorhandensein die Präzipitation der durch Reaktion zwischen M1 und M2 gebildeten Verbindung M1-M2 verhindert und es ermöglicht, eine Lösung zu erhalten, deren Konzentration an Verbindung M1-M2 höher ist als die, die durch die Präzipitationskonstante der Verbindung M1-M2 in Abwesenheit von Molekülen der dritten Art M3 bestimmt wird, und
- wobei die Moleküle der dritten Art, die für die Definition von Addukten in Lösung verantwortlich sind, wobei die erste oder zweite Art von Molekülen organolösliche Moleküle mit mindestens einer Koordinationsgruppe sind, die eine Verbindung sein kann, die zu einer der folgenden Familien gehört:
- Amine, die 1-Octylamin oder 1-Pentylamin sein können;
- Carbonsäuren;
- Ester;
- Siloxane;
- Phosphine;
- Moleküle mit einem koordinierenden n-System, das 3-Hexylthiophen sein kann.

2. Stabile Lösung aus molekularem Material nach Anspruch 1, **dadurch gekennzeichnet, dass** sie ferner umfasst:
- eine vierte Art von Molekülen (M4), die ein Tensid oder Lösungsmittel sein können, die in der Lage sind, sich mit der ersten und zweiten Art von organischen oder metallorganischen Molekülen (M1, M2) zu assoziieren;
- wobei die Moleküle (M4) der vierten Art organolösliche Moleküle mit mindestens einer koordinierenden Gruppe sind, die eine Verbindung sein kann, die zu einer der folgenden Familien gehört:
- Amine, die 1-Octylamin oder 1-Pentylamin sein können;
- Carbonsäuren;
- Ester;
- Siloxane;
- Phosphine;
- Moleküle mit einem koordinierenden n-System, das 3-Hexylthiophen sein kann.

3. Stabile Lösung aus molekularem Material nach einem der Ansprüche 1 oder 2, **dadurch gekennzeichnet, dass** die erste oder zweite Art von Molekülen organische oder metallorganische Moleküle, organische oder metallorganische Molekülsalze sind.

4. Stabile Lösung aus molekularem Material nach einem der vorherigen Ansprüche, **dadurch gekennzeichnet, dass** die erste und/oder zweite Art von Molekülen oder Ionen (M1, M2) ein organisches oder organometallisches Donor-Molekül ist, das von der folgenden Art sein kann:
- Derivat von Tetrathiafulvalen [TTF];
- Übergangsmetallkomplexe;
- Metallkomplexe mit Bisdithiolen-Liganden: M(Dmit)₂ in neutralem Zustand oder mit M geladen: Ni, Pd, Pt und Dmit: 4,5-Dimercapto-1,3-Dithiol-2-thion;
- Metallkomplexe mit Liganden vom Typ TTF-Bisdithiolen wie Tmdt: M(Tmdt)₂ in neutralem Zustand oder geladen mit Tmdt: Trimethylentertrathiafulvalendithiolat und M: Ni, Pd, Pt;
- Metallkomplexe mit Dcbdt-Liganden: M(Dcbdt)₂, in neutralem Zustand oder geladen mit Dcbdt : Dicyanobenzenedithiolat und M: Ni, Pd, Pt;
- Perylen.

5. Stabile Lösung aus molekularem Material nach einem der vorherigen Ansprüche, **dadurch gekennzeichnet, dass** die erste und/oder zweite Art von Molekülen (M1, M2) ein organisches oder metallorganisches Molekül vom Akzeptortyp ist, das von dem folgenden Typ sein kann:
- ein Derivat von TCNQ: Tetracyano-p-chinodimethan;
- TNAP: 11,11,12,12,-Tetracyanoaphtho-2,6-chinodimethan;
- DCNQI: Dicyanochinondiimin.

6. Stabile Lösung aus molekularem Material nach einem der vorherigen Ansprüche, **dadurch gekennzeichnet, dass**, wenn nur eine erste Art oder eine zweite Art von der Art eines organischen oder metallorganischen Moleküls ist, die andere Art von der ionischen Art sein und eines der folgenden Anionen sein kann: Hexafluorophosphate, Triiodid, Chlorid, Bromid, Arsenate, Antimonate.

7. Stabile Lösung aus molekularem Material nach einem der vorherigen Ansprüche, **dadurch gekennzeichnet, dass**, wenn nur eine erste Art oder eine zweite Art von der Art eines organischen oder metallorganischen Moleküls ist, die andere Art von der ionischen Art sein kann und eines der folgenden Kationen sein kann: Na⁺ (Natrium), K⁺ (Kalium) oder ein Übergangsmetallkation.

8. Stabile Lösung aus molekularem Material nach einem der vorherigen Ansprüche, **dadurch gekennzeichnet, dass**
- die Moleküle der ersten Art TTF-Moleküle sind;
- die Moleküle der zweiten Art TCNQ-Moleküle sind;
- die Moleküle der dritten Art Aminmoleküle mit langen Kohlenstoffketten sind.

9. Stabile Lösung aus molekularem Material nach einem der vorherigen Ansprüche, **dadurch gekennzeichnet, dass**
- die Moleküle der ersten Art TTF-Moleküle sind;
- die Moleküle der zweiten Art TCNQ-Moleküle sind;
- die Moleküle der dritten Art Aminmoleküle mit langen Kohlenstoffketten sind.

10. Verfahren zur Herstellung einer stabilen Lösung aus molekularem Material nach einem der vorherigen Ansprüche, **dadurch gekennzeichnet, dass** es die folgende Mischung umfasst:
- eine erste Art von organischen oder metallorganischen Molekülen (M1) und eine zweite Art von organischen oder metallorganischen Molekülen oder Ionen (M2), wobei die erste Art mindestens Gruppierungen aufweist, die eine elektronische Verlagerung ermöglichen und in Gegenwart von mindestens einer dritten Art von Molekülen (M3) einen molekularen Kristall bilden können;
- wobei die Moleküle der dritten Art, die für die Definition von Addukten in Lösung verantwortlich sind, wobei die erste oder zweite Art von Molekülen organolösliche Moleküle mit mindestens einer Koordinationsgruppe sind, die eine Verbindung sein kann, die zu einer der folgenden Familien gehört:
- Amine, die 1-Octylamin oder 1-Pentylamin sein können;
- Carbonsäuren;
- Ester;
- Siloxane;
- Phosphine;
- Moleküle, die ein koordinierendes π-System besitzen, das 3-Hexylthiophen sein kann und
dessen Vorhandensein die Präzipitation der durch Reaktion zwischen M1 und M2 gebildeten Verbindung M1-M2 verhindert und es ermöglicht, eine Lösung zu erhalten, deren Konzentration an Verbindung M1-M2 höher ist als die, die durch die Präzipitationskonstante der Verbindung M1-M2 in Abwesenheit von Molekülen der dritten Art M3 bestimmt wird.

11. Verfahren zur Herstellung einer stabilen Lösung aus molekularem Material, das molekulare Materialien nach Anspruch 10 umfasst, **dadurch gekennzeichnet, dass** es das Hinzufügen einer dritten Art von Molekülen (M3) und einer vierten Art von Molekülen (M4) umfasst, die ein Tensid oder ein Lösungsmittel sein können, das in der Lage ist, sich mit der ersten und zweiten Art von organischen oder metallorganischen Molekülen (M1, M2) zu assoziieren;
- wobei die Moleküle (M4) der vierten Art organolösliche Moleküle mit mindestens einer koordinierenden Gruppe sind, die eine Verbindung sein kann, die zu einer der folgenden Familien gehört:
- Amine, die 1-Octylamin oder 1-Pentylamin sein können;
- Carbonsäuren;
- Ester;
- Siloxane;
- Phosphine;
- Moleküle mit einem koordinierenden π-System, das 3-Hexylthiophen sein kann.

12. Verfahren zur Herstellung einer stabilen Lösung aus molekularem Material nach einem der vorherigen Ansprüche, **dadurch gekennzeichnet, dass** es die folgenden Schritte umfasst:
- das Mischen von TTF-Molekülen und langkettigen kohlenstoffhaltigen Aminmolekülen in einem organischen Lösungsmittel, um ein Primärgemisch zu erlangen;
- das Hinzufügen von TCNQ-Molekülen in einem organischen Lösungsmittel zu dem Primärgemisch.

13. Verfahren zur Herstellung einer stabilen Lösung aus molekularem Material nach einem der vorherigen Ansprüche, **dadurch gekennzeichnet, dass** es die folgenden Schritte umfasst:
- das Mischen von TTF₃(BF₄₎₂-Molekülen und langkettigen kohlenstoffhaltigen Aminmolekülen in einem organischen Lösungsmittel, um ein Primärgemisch zu erlangen;
- das Hinzufügen von Molekülen von [(*n*-C₄H₉)₄N][Ni(Dmit)₂] in einem organischen Lösungsmittel zu dem Primärgemisch.

14. Verfahren zur Herstellung eines Nanopartikelpulvers aus Molekularmaterial, **dadurch gekennzeichnet, dass** es Folgendes umfasst:
- die Verfahrensschritte zum Erzielen einer stabilen Lösung nach einem der Ansprüche 10 bis 13;
- einen Trocknungsschritt der stabilen Lösung.

15. Verfahren zur Herstellung einer Folie auf Grundlage von molekularem Material, **dadurch gekennzeichnet, dass** sie Folgendes umfasst:
- die Verfahrensschritte zum Erzielen einer stabilen Lösung nach einem der Ansprüche 10 bis 13;
- das Abscheiden der stabilen Lösung auf einem Träger;
- das Trocknen der stabilen Lösung auf dem Träger.

16. Verfahren zur Herstellung eines Verbundmaterials auf der Grundlage von molekularem Material, **dadurch gekennzeichnet, dass** es Folgendes umfasst:
- die Verfahrensschritte zum Erzielen einer stabilen Lösung nach einem der Ansprüche 10 bis 13;
- das Mischen der stabilen Lösung mit einer organischen oder anorganischen Matrix.

17. Verfahren zur Herstellung eines Verbundmaterials auf der Grundlage von molekularem Material, **dadurch gekennzeichnet, dass** es Folgendes umfasst:
- die Schritte der Herstellung eines Nanopartikelpulvers aus molekularem Material nach Anspruch 15;
- das Mischen des Pulvers mit einer organischen oder anorganischen Matrix.

18. Verwendung einer stabilen Lösung aus molekularem Material nach einem der Ansprüche 1 bis 9 zur Herstellung eines elektromagnetisch absorbierenden Materials.

19. Verwendung einer stabilen Lösung aus molekularem Material nach einem der Ansprüche 1 bis 9 zur Herstellung eines aktiven Materials für die Elektronik.

20. Verwendung einer stabilen Lösung aus molekularem Material nach Anspruch 19 zur Herstellung von Leiterbahnen oder Transistoren oder lichtemittierenden Dioden.

## Claims

1. Stable solution of molecular material, comprising:
- a first type of organic or organometallic molecules (M1);
- the first type at least having groups allowing electron delocalization;
- a third type of molecules (M3) that may be a surfactant or a solvent, **characterized in that** it also comprises a second type of organic or organometallic molecules or of ions (M2);
- said third type being capable of combining with the first or the second type of molecules or of ions (M1, M2) to form an adduct in solution whose presence prevents the precipitation of the compound M1-M2 formed by reaction between M1 and M2 and makes it possible to obtain a solution whose concentration of compound M1-M2 is greater than that governed by the precipitation constant of the compound M1-M2 in the absence of molecules of the third type M3, and
- the third type of molecules charged with defining adducts in solution with the first or second types of molecules being organosoluble molecules bearing at least one coordinating group, which may be a compound belonging to one of the following families:
- amines, which may be 1-octylamine or 1-pentylamine;
- carboxylic acids;
- esters;
- siloxanes;
- phosphines;
- molecules bearing a *π* coordinating system, which may be 3-hexylthiophene.

2. Stable solution of molecular material according to claim 1, **characterized in that** it further comprises:
- a fourth type of molecules (M4), which may be a surfactant or a solvent, capable of combining with the first and the second types of organic or organometallic molecules (M1, M2);
- the molecules (M4) of the fourth type being organosoluble molecules bearing at least one coordinating group, which may be a compound belonging to one of the following families:
- amines, which may be 1-octylamine or 1-pentylamine;
- carboxylic acids;
- esters;
- siloxanes;
- phosphines;
- molecules bearing a *π* coordinating system, which may be 3-hexylthiophene.

3. Stable solution of molecular material according to one of claims 1 or 2, **characterized in that** the first or second type of molecules are organic or organometallic molecules, organic or organometallic molecular salts.

4. Stable solution of molecular material according to one of the preceding claims, **characterized in that** the first and/or second type of molecules or of ions (M1, M2) is an organic or organometallic molecule of donor type, which may be of the type such as:
- a tetrathiafulvalene [TTF] derivative;
- transition metal complexes;
- complexes of metals with bisdithiolene ligands: M(dmit)₂ in neutral or charged state with M: Ni, Pd, Pt and dmit: 4,5-dimercapto-1,3-dithiole-2-thione;
- complexes of metals with ligands of TTF-bisdithiolene type such as tmdt: M(tmdt)₂ in neutral or charged state with tmdt: trimethylenetetrathiafulvalene-dithiolate and M: Ni, Pd, Pt;
- complexes of metals with dcbdt ligands: M(dcbdt)₂, in neutral or charged state with dcbdt: dicyanobenzenedithiolate and M: Ni, Pd, Pt;
- perylene.

5. Stable solution of molecular material according to one of the preceding claims, **characterized in that** the first and/or second type of molecules (M1, M2) is an organic or organometallic molecule of acceptor type, which may be of the type such as:
- a TCNQ derivative: tetracyano-p-quinodimethane;
- TNAP: 11,11,12,12-tetracyanonaphtho-2,6-quinodimethane;
- DCNQI: dicyanoquinonediimine.

6. Stable solution of molecular material according to one of the preceding claims, **characterized in that**, when only one first type or second type is of the organic or organometallic molecules type, the other type may be of ionic type and may be included among one of the following anions: hexafluorophosphates, triiodide, chloride, bromide, arseniates, antimoniates.

7. Stable solution of molecular material according to one of the preceding claims, **characterized in that**, when only one first type or second type is of organic or organometallic molecule type, the other type may be of ionic type and may be included among one of the following cations: Na⁺ (sodium), K⁺ (potassium) or a transition metal cation.

8. Stable solution of molecular material according to one of the preceding claims, **characterized in that**:
- the molecules of the first type are TTF molecules;
- the molecules of the second type are TCNQ molecules;
- the molecules of the third type are long-carbon-chain amine molecules.

9. Stable solution of molecular material according to one of the preceding claims, **characterized in that**:
- the molecules of the first type are TTF molecules;
- the molecules of the second type are TCNQ molecules;
- the molecules of the third type are long-carbon-chain amine molecules.

10. Process for preparing a stable solution of molecular material according to one of the preceding claims, **characterized in that** it includes the mixing:
- of a first type of organic or organometallic molecules (M1) and of a second type of organic or organometallic molecules or of ions (M2), the first type at least having groups allowing electron delocalization, and being capable of forming a molecular crystal, in the presence of at least a third type of molecules (M3);
- the third type of molecules charged with defining adducts in solution with the first or second types of molecules being organosoluble molecules bearing at least one coordinating group, which may be a compound belonging to one of the following families:
- amines, which may be 1-octylamine or 1-pentylamine;
- carboxylic acids;
- esters;
- siloxanes;
- phosphines;
- molecules bearing a *π* coordinating system, which may be 3-hexylthiophene and
whose presence prevents the precipitation of the compound M1-M2 formed by reaction between M1 and M2 and makes it possible to obtain a solution whose concentration of compound M1-M2 is greater than that governed by the precipitation constant of the compound M1-M2 in the absence of molecules of the third type M3.

11. Process for preparing a stable solution of molecular material comprising molecular materials according to Claim 10, **characterized in that** it comprises the addition of a third type of molecules (M3) and of a fourth type of molecules (M4) which may be a surfactant or a solvent, capable of combining with the first and the second types of organic or organometallic molecules (M1, M2);
- the molecules (M4) of the fourth type being organosoluble molecules bearing at least one coordinating group, which may be a compound belonging to one of the following families:
- amines, which may be 1-octylamine or 1-pentylamine;
- carboxylic acids;
- esters;
- siloxanes;
- phosphines;
- molecules bearing a *π* coordinating system, which may be 3-hexylthiophene.

12. Process for preparing a stable solution of molecular material according to one of the preceding claims, **characterized in that** it comprises the following steps:
- the mixing of TTF molecules and of long-carbon-chain amine molecules in an organic solvent, so as to obtain a primary mixture;
- the addition of TCNQ molecules in an organic solvent to the primary mixture.

13. Process for preparing a stable solution of molecular material according to one of the preceding claims, **characterized in that** it comprises the following steps:
- the mixing of TTF₃(BF₄)₂ molecules and of long-carbon-chain amine molecules in an organic solvent, so as to obtain a primary mixture;
- the addition of [(*n*-C₄H₉)₄N][Ni(dmit)₂] molecules in an organic solvent to the primary mixture.

14. Process for preparing a nanoparticulate powder of molecular material, **characterized in that** it comprises:
- the steps of the process for preparing a stable solution according to one of claims 10 to 13;
- a step of drying the stable solution.

15. Process for manufacturing a film based on molecular material, **characterized in that** it comprises:
- the steps of the process for producing a stable solution according to one of claims 10 to 13;
- the deposition of said stable solution onto a support;
- the drying of said stable solution on said support.

16. Process for manufacturing a composite material based on molecular material, **characterized in that** it comprises:
- the steps of the process for manufacturing a stable solution according to one of claims 10 to 13;
- the mixing of said stable solution with an organic or inorganic matrix.

17. Process for manufacturing a composite material based on molecular material, **characterized in that** it comprises:
- the steps of preparing a nanoparticulate powder of molecular material according to claim 15;
- the mixing of said powder with an organic or inorganic matrix.

18. Use of a stable solution of molecular material according to one of claims 1 to 9, for the preparation of an electromagnetic absorbent material.

19. Use of a stable solution of molecular material according to one of claims 1 to 9, for the preparation of an active material for electronics.

20. Use of a stable solution of molecular material according to claim 19, for the preparation of a conductive track or a transistor or a light-emitting diode.
